# EUROPEAN PATENT APPLICATION

(11) **EP 3 858 806 A1**
(43) Date of publication of application: **04.08.2021**
(21) Application number: 20154277.6
(22) Date of filing: 29.01.2020
(51) Int. Cl.: C07C 1/24, C07D 301/10, B01J 23/30, B01J 23/50, B01J 23/66, B01J 21/04, B01J 21/08, B01J 21/06, B01J 27/053, B01J 35/10

(54) **PROCESS FOR PREPARING ETHYLENE OXIDE FROM ETHANOL**

(71) Applicant: Clariant International Ltd, 4132 Muttenz (CH); Universität Stuttgart, 70174 Stuttgart (DE)
(72) Inventor: BRAND, Stefan, 69493 Hirschberg-Leutershausesn (DE); FRANKE, Oliver, 81671 München (DE); MESTL, Gerhard, 80935 Muenchen (DE); OTTERSTAETTER, Robin, 86438 Kissing (DE); WACHSEN, Olaf, 84518 Garching (DE); KLEMM, Elias, 79569 Stuttgart (DE); HIMMELMANN, Robin, 79569 Stuttgart (DE)
(74) Representative: Kuba, Stefan

(57) **Abstract**

The invention relates to a catalyst arrangement for preparation of ethylene oxide from ethanol, comprising a first catalyst which is suitable for dehydration of ethanol to ethylene and is in fluid connection to a second catalyst suitable for oxidation of ethylene to ethylene oxide with oxygen, characterized in that the first catalyst is an acidic catalyst having an overall density of acidic sites of more than 1 µmol/m². The invention also relates to a process for preparing ethylene oxide from ethanol, comprising the steps of: a) contacting ethanol with a first catalyst suitable for dehydration of ethanol to ethylene to obtain ethylene,
b) contacting the ethylene from step a) with a second catalyst suitable for oxidation of ethylene to ethylene oxide with oxygen, characterized in that the first catalyst is an acidic catalyst having an overall density of acidic sites of more than 1 µmol/m². The first catalyst used is preferably sulfated zirconium dioxide or tungstosilicic acid on an SiO₂ support.

## Description

The invention relates to a process for preparing ethylene oxide from ethanol, comprising the steps of: a) contacting ethanol with a first catalyst suitable for dehydration of ethanol to ethylene to obtain ethylene, b) contacting the ethylene from step a) with a second catalyst suitable for oxidation of ethylene to ethylene oxide with oxygen, characterized in that the first catalyst is an acidic catalyst having a density of acidic sites of more than one µmol/m².

Ethanol can be obtained by the fermentation of biomass, for example of starch- or sugar-containing plants. It is desirable here to use cellulose-containing waste materials as a renewable and inedible source, called 2nd generation bioethanol (S. Kim, B. E. Dale, Biomass Bioenergy, 26 (2004) 361). The production costs of ethanol have significantly decreased in the last decade, in particular as a result of continuous industrial progress (R. Wooley, M. Ruth, D. Glassner, J. Sheehan, Biotechnol. Prog., 15 (1999) 794). The effect of the low cost and high production capacities has been that bioethanol is mixed into gasoline in high proportions, reducing dependence on fossil sources. However, bioethanol is not just a sustainable fuel but also a versatile chemical starting material; for instance, ethanol can enter into various kinds of reactions and be converted, for example, to acetaldehyde (oxidation), ethyl acetate (oxidation and subsequent esterification), synthesis gas (gasification) or ethylene (dehydration). The latter can be converted to ethylene oxide by an epoxidation (oxidation) with oxygen in an industrially very important reaction.

Ethylene oxide is the most important and also the most valuable of the various molecules that can be obtained from ethanol. It is used for the preparation of ethylene glycol, polyethylene glycol and polyester fibres, and also plastic from polyethylene terephthalates (PET) (Z.L. Arsenijević, B.V. Grbić, N.D. Radić, B. Grbav̌cić, Chem. Eng. J. 116 (2006) 173-178). Nowadays, ethylene oxide is obtained almost exclusively from ethylene from fossil sources. The production of ethylene oxide is therefore not sustainable and highly dependent on the cost of fossil sources (K. Weissermel, H-J Arpe, Ind. Org. Chem. (4th ed.) (2003) 145-148). The idea of using ethylene oxide from the sustainable raw material ethanol has therefore gained significant attention. The direct oxidation of ethanol to ethylene oxide with alumina-supported silver, copper and gold catalysts has been reported (M.J. Lippits, B.E. Nieuwenhuys, Catal. Today, 154 (2010) 127-132; M.J. Lippits, B.E. Nieuwenhuys, J. Catal., 274 (2010) 142-149). At present, however, these catalysts for direct oxidation do not reach the yield necessary for industrial use. However, the 2-stage process consisting of dehydration of ethanol and the subsequent oxidation of the ethylene thus obtained is promising for industrial use. In this 2-stage process, acidic catalysts are used in the 1st step for dehydration of the ethanol. The ethylene thus obtained is then converted to ethylene oxide in a 2nd, separate step with the customary silver-containing oxidation catalysts as also used in the oxidation of ethylene from fossil sources.

US 20190100476 A1 discloses a process for preparing ethylene from an ethanol starting material, comprising a step of subjecting the ethanol starting material to a dehydration reaction in the presence of a supported heteropolyacid salt catalyst, wherein the supported heteropolyacid salt catalyst is prepared from a supported heteropolyacid catalyst comprising a support and a heteropolyacid present on the support with a weight ratio of the heteropolyacid to the support within a range from 0.1:1 to 2.5:1; and wherein the supported heteropolyacid salt catalyst comprises a support and a heteropolyacid salt compound which is present on the support and is represented by a formula selected from the group consisting of: (M^{a}ₙH₄₋ₙ)X^{a}M^{d}₁₂O₄₀ formula 1, (M^{b}_{q}H_{3-q})X^{b}M^{e}₁₂O₄₀ formula 2 and (M^{c}ₚH₆₋ₚ)X^{c}M^{f}₁₈O₆₂ formula 3, in which: M^{a}, M^{b} and M^{c} are independently selected from the group consisting of Cu, Ag, Au, Zn, Cd and Hg; X^{a} is selected from the group consisting of Si and Ge; X^{b} and X^{c} are independently selected from the group consisting of P and As; M^{d}, M^{e} and M^{f} are independently selected from the group consisting of Mo and W; n is an integer in the range from 1 to 4; q is an integer in the range from 1 to 3; and p is an integer in the range from 1 to 6.

US 2019/0100476 A1 relates to a process for preparing ethylene from methanol as starting material, comprising the step of subjecting ethanol starting material to a dehydration reaction in the presence of a supported heteropolyacid salt catalyst. The supported heteropolyacid salt catalyst comprises a support and a heteropolyacid salt compound present on the support.

Examples of silver catalysts that can be employed in the preparation of ethylene oxide by the oxidation of ethylene at elevated temperatures in the presence of oxygen can be found, for example, in US 3878126A and US 4774222A.

WO 2014 018474 A1 discloses a process for preparing ethylene oxide, comprising: providing one or more charge components, wherein the one or more charge components comprise at least ethylene that has been obtained by dehydration of ethanol; contacting the one or more charge components with a desulfurization catalyst comprising a support having high surface area and an amount of silver, where at least 20% of the silver is in the form of oxidized silver; and contacting the one or more charge components with a silver-containing epoxidation catalyst disposed in an ethylene oxide reactor, in order to form a reaction gas comprising ethylene oxide.

BR 8200311A discloses a process wherein the conversion of ethanol to ethylene oxide is performed in two steps within a single reactor. The reaction step of dehydrating the ethanol to ethylene is effected by the contacting of the ethanol mixed with diluent gases and recirculation gases with the catalytic fluidized bed present within the reactor housing and outside the connected reaction tubes of the tube bundle. Within these reaction tubes is a rigid bed of an oxidation catalyst. After being passed through the fluidized bed, the gases, now containing ethylene and a stream of oxygen, or air, are introduced into the upper portion of the reactor and guided into the interior of the reaction tubes. As a result of the contact with the catalytic bed, the ethylene is oxidized to ethylene oxide. The heat which is released in the oxidation is guided through the reaction tubes to the catalytic fluidized bed. Performing of the two reaction steps within a single reactor combines the advantageous effects of both. On the one hand, the heat that arises from the oxidation reaction supplies the endothermic ethanol dehydration reaction to be performed with heat, which leads to saving of fuel/oil or to saving of another energy source. On the other hand, the fluidized bed in which the dehydration reaction takes place is a very good absorber of heat, which is required for the control of the oxidation reaction, which leads to saving of thermal exchange surfaces and to saving of cooling fluids that are used in other processes.

The inventors propose an improved catalyst arrangement and an improved 2-stage process for preparing ethylene oxide from ethanol. It is an object of the invention to provide a novel catalyst arrangement and a novel improved 2-stage process for preparing ethylene oxide from ethanol. The novel catalyst arrangement allows a simplification of the existing 2-stage processes for preparing ethylene oxide from ethanol. As well as the simplification of the process, the novel catalyst arrangement permits elevated yields, greater economic viability of operation and improved stability.

The object is achieved by a catalyst arrangement for preparation of ethylene oxide from ethanol, comprising a first catalyst [1] which is suitable for dehydration of ethanol to ethylene and is in fluid connection to a second catalyst [2] suitable for oxidation of ethylene to ethylene oxide with oxygen, characterized in that the first catalyst [1] is an acidic catalyst having an overall density of acidic sites of more than 1 µmol/m².

The object is also achieved by a process for preparing ethylene oxide from ethanol, wherein a reactant gas stream containing oxygen and ethanol is guided through a catalyst arrangement according to the invention in order to obtain a product gas stream.

Alternatively, the process according to the invention may also be formulated as a process for preparing ethylene oxide from ethanol, comprising the steps of:
contacting ethanol with a first catalyst [1] suitable for dehydration of ethanol to ethylene to obtain ethylene,
contacting the ethylene from step a) with a second catalyst [2] suitable for oxidation of ethylene to ethylene oxide with oxygen,
characterized in that the first catalyst [1] is an acidic catalyst having a density of acidic sites of more than 1 µmol/m².
The invention also relates to the use of the catalyst arrangement of the invention for preparation of ethylene oxide from ethanol.

The process according to the invention can be performed at a temperature in the region of the first catalyst [1] between 100 and 300°C, preferably between 180 and 280°C, and/or at a temperature in the region of the second catalyst [2] between 150°C and 300°C, preferably between 210°C and 280°C.

The first catalyst [1] may be any acidic catalyst that catalyses the dehydration of ethanol to ethylene and has an overall density of acidic sites (overall acid density), i.e. of Lewis- and Bronsted-acidic sites, of greater than 1 µmol/m². For example, this first catalyst [1] may be an acidic metal oxide, such as a heteropolytungstic acid, preferably tungstosilicic acid (H₄[Si(W₁₂O₄₀)]· 26H₂O), preferably supported on an inert support, for example SiO₂, Al₂O₃, or ZrO₂. A further preferred acidic metal oxide is sulfated metal oxide, such as sulfated zirconium dioxide. The acidic metal oxide may suitably be blended with a binder in order to form catalyst bodies therefrom.

Preferably, the first catalyst [1] in the catalyst arrangement according to the invention, or in the performance of the process according to the invention, has an overall density of acidic sites of more than 1.2 µmol/m², preferably more than 1.5 µmol/m². The first catalyst [1] here may be a tungsten-containing heteropolyacid or a sulfated metal oxide, especially sulfated zirconium dioxide or a tungstosilicic acid on an SiO₂ support.

The acidic sites of the acidic first catalyst [1] may be either Lewis-acidic sites, as arise, for example, from defects, or Bronsted-acidic sites in the form of OH groups on the surface.

The density or concentration of the active sites in the first catalyst [1] according to the invention is more than 1 µmol/m² based on the surface area of the catalyst, as ascertained by the BET method. It is preferable that the density of acidic sites of the first catalyst [1] is greater than 1.2 µmol/m², preferably greater than 1.5 µmol/m².

The second catalyst [2] may be any catalyst that catalyses the oxidation of ethene to ethylene oxide. Preferably, the second catalyst [2] in the catalyst arrangement according to the invention, or in the performance of the process according to the invention, is a catalyst containing or consisting of Ag on an alumina support.

The catalyst arrangement according to the invention that is suitable for performance of the process according to the invention is configured such that it has an inlet [6] in fluid connection to the first catalyst [1] and an outlet [7] in fluid connection to the second catalyst [2]. Such a catalyst arrangement is configured such that reactant gases can be introduced into the inlet [6] and these can then flow through the first catalyst [1] in order to react there. In addition, such a catalyst arrangement is configured such that the reaction gases that exit from the region of the first catalyst [1] can flow through the second catalyst [2] in order then to be able to be guided out again at the outlet [7] as product gases. What is meant here by the existence of a fluid connection is that gases can flow between the connected articles irrespective of whether further devices, for example purification means or means of altering the temperature of the gases, are mounted between the connected articles. Preferably, the fluid connection is loss-free, meaning that the gas being guided is guided completely from one article to another.

The invention further relates to a process for preparing ethylene oxide from ethanol, wherein a gas stream containing oxygen and ethanol is guided through a catalyst arrangement according to the invention in order to obtain a product gas stream. A raw ethylene oxide stream [8] can be removed here ("separated") from the product gas stream by a separator [3] and the remaining product gas stream [4] can be fed back into the catalyst arrangement upstream of the first catalyst [1] into the reactant gas stream ("recycling"). It is also possible that the reactant gas stream already contains ethylene and/or diluent gases such as nitrogen. In a preferred embodiment, the reactant gas stream contains 20% by volume to 40% by volume of ethene and the product gas stream is recycled, with replacement of the ethene converted by the equivalent amount of ethanol.

In a preferred configuration of the catalyst arrangement which is suitable for performance of the process according to the invention, the first catalyst [1] and the second catalyst [2] are preferably in direct fluid connection, meaning that the gas flow between the two catalysts does not lead through further means of gas treatment, for example means of heating or means of purifying the gases. What is advantageous about this configuration is that it is possible to dispense, for example, with means of purifying the ethylene-containing reaction gas that leaves the region of the first catalyst [1] in gaseous form, which constitutes a simplification of the catalyst arrangement.

In a preferred configuration of the catalyst arrangement which is suitable for performance of the process according to the invention, both catalysts are accommodated in a single reactor [5], which can be configured as a tube. It is possible here for a multitude of reactor tubes configured in accordance with the invention to be accommodated in a single housing, as is the case in a shell and tube reactor.

In a further preferred configuration of the catalyst arrangement which is suitable for performance of the process according to the invention, the temperature in the region of the first catalyst [1] can be controlled independently of the temperature in the region of the second catalyst [2], meaning that the two catalysts are thermally decoupled. This can be achieved, for example, in that a means of temperature control is mounted in the region of the first catalyst [1] and a further means of temperature control is mounted in the region of the second catalyst [2], each of which can be controlled independently of one another. A means of temperature control is, for example, a salt bath, an oven or a heat exchanger.

In the performance of the process according to the invention, it is possible to mechanically stabilize the two catalysts, for example in a reactor, by including an intermediate layer of inert bodies between the two catalysts. Alternatively, it is possible that the two catalysts directly follow one another or are touching, i.e. in contact with one another.

The first catalyst [1] which is suitable for dehydration of ethanol to ethylene may advantageously be configured as a fixed bed catalyst, which constitutes a significant simplification of the process, since the operating of a fluidized bed is complex from a chemical engineering point of view and additionally represents a high level of mechanical stress on the catalyst. The fixed bed catalyst is in the form of powder or of catalyst bodies that do not move in the gas stream, as is the case in a fluidized catalyst bed.

Preferably, in the catalyst arrangement according to the invention, or in the performance of the process according to the invention, both the first catalyst [1] and the second catalyst [2] are in the form of a fixed bed catalyst in the reactor. The catalysts here may be in powder form, but preferably consist of catalyst bodies that can assume various shapes and sizes. The catalyst bodies may be configured, for example, as pellets, spheres or rings in the order of magnitude between 1 mm and 10 cm. The catalyst bodies then form the fixed bed. Alternatively, the respective catalyst may in each case also take the form of an active layer on a shaped honeycomb body, in which case the individual shaped honeycomb body forms the fixed bed.

### Methods

### BET

The samples were analysed on an Autosorb 3B from Quantachrome. The samples were baked at 250°C under reduced pressure (p < 10⁻⁶ bar) for at least 16 h. The baked sample was weighed (catalyst surface area always based on dry weight) and analysed by means of nitrogen adsorption at 77 K. In the case of micro- and mesoporous materials, correspondingly configured measurement programs were used in order to determine pore volumes and diameters.

### Acidic site density

The acidic site density was determined by means of ammonia loading and MAS-NMR; the method is described in detail in ChemCatChem 2016, 8, 2031 - 2036 DOI:10.1002/cctc.201600372.

### Process procedure in separation of the catalysts by quartz wool

The catalysts were layered one on top of another in a reactor, separated by quartz glass wool, in such a way that the reaction feed flowed over the dehydration catalyst first. Prior to performance of the measurements, the reactor was brought to the reaction temperature specified under nitrogen. The reaction was started by guiding the desired gas stream over the catalysts, for instance at standard pressure.

### Performance of the reactions with separation of the catalysts by heated pipelines

The catalysts were introduced into two different reactors, separated by a heated pipeline. Prior to performance of the measurements, the reactors were brought to the reaction temperature specified under nitrogen. The reaction was started by guiding the desired gas stream at about standard pressure first to the reactor charged with the respective dehydration catalyst. After a reaction time of about one hour, the second reactor charged with the silver catalyst was connected.

### Examples

### 1. Preparation of the first catalyst

### Catalyst A1: tungstosilicic acid on SiO₂

The shaped catalyst bodies of the first catalyst layer for dehydration of the ethanol were produced by impregnation by the incipient wetness method, wherein tungstosilicic acid was applied to an SiO₂ support.

For the catalyst synthesis, a magnetic stirrer of the IKAMAG RET type from IKA and a muffle furnace of the N11/R type from Nabertherm were used; these were purged with synthetic air (14.5 l/h) or nitrogen (45 l/h) as specified. The SiO₂ support used was Aerosil 200 V from Degussa, which was calcined in the muffle furnace at 550°C for 16 hours prior to use.

For the impregnation solution, 25 g of the tungstosilicic acid hydrate (Sigma-Aldrich, purity > 99.9%) were dissolved in 600 ml of water at room temperature while stirring. 50 g of the catalyst support prepared was added in powder form to the colourless solution, under constant stirring with a magnetic stirrer. The resultant colourless to white gel was stirred at room temperature for 65 h and then transferred to a calcining dish. The gel was heated to 70°C in a muffle furnace with a temperature ramp of 1°C/min, and this temperature was maintained for 48 h. This was followed by further heating to 96°C at 1°C/min and holding the temperature for 21 h, before heating to 120°C at 1°C/min. The final temperature was maintained for 2 h, before the catalyst (catalyst A) was cooled down to room temperature. While all the temperature ramps and hold times were being performed in the muffle furnace, synthetic air flowed through it permanently.

**Table 1: analysis results for catalyst A by comparison with the Aerosil 200V support**

| Sample | N₂ BET [m²/g] | Micropore vol. [cc/g] | Micropore surface area [m²/g] |
|---|---|---|---|
| Aerosil 200V | 167.6 | 0.005 | 15.1 |
| Catalyst A1 | 139.2 | 0.014 | 33.1 |

### Catalyst A2: sulfated ZrO₂

A 15% by weight solution of zirconium(IV) oxynitrate in demineralized water was stirred at 25°C for 2 h. During this time, the pH was kept between pH = 9 to 10 by the addition of a 25% NH₃ solution (aqueous). Verification was by means of a universal indicator paper (pH 1 to 14). The resultant suspension was aged for 2 h, then filtered through a white-band filter paper and washed with demineralized water to free it of nitrate. Testing with nitrate/nitrite test strips (amount of nitrate < 10 mg/l). The white powder was dried in a calcining dish at 110°C in a muffle furnace under air (14.5 l/h) for 24 h. The resultant white solid was suspended in 0.5 molar sulfuric acid (2.68 ml/g of support). Subsequently, the suspension was heated to 80°C in an oil bath and concentrated by evaporation for complete drying. The resultant white solid was then dried again in a muffle furnace at 110°C for 3 h and subsequently heated up to 500°C at 2 K/min. The catalyst was calcined at 500°C for 3 h. Synthetic air flowed continuously through the muffle furnace at 14.5 l/h.

**Table 2: acidities of the first catalyst**

| **Sample** | **N₂ BET [m²/g]** | **Brønsted acidity [mmol/g]** | **Lewis acidity [mmol/g]** | **Total acid density [mmol/g]** | **Total acid density [µmol/m²]** |
|---|---|---|---|---|---|
| Catalyst A1 | 139.2 | 0.365 | 0 | 0.365 | 2.62 |
| Catalyst A2 | 134 | 0.056 | 0.102 | 0.158 | 1.18 |
| H-ZSM-5 (Si/Al=30) | 408 | 0.343 | 0 | 0.343 | 0.84 |
| H-ZSM-5 (Si/Al=45) | 435 | 0.247 | 0 | 0.247 | 0.57 |

### 2. Preparation of the second catalyst

### Catalysts B1 and B2: silver on the alumina support

The shaped catalyst bodies of the second catalyst layer, for the epoxidation of the ethylene formed in situ, were prepared by impregnation by the incipient wetness method, with application of silver to the alumina support.

For the catalyst synthesis, a magnetic stirrer of the IKAMAG RET type from IKA and a muffle furnace of the N11/R type from Nabertherm were used; these were purged with synthetic air (14.5 l/h) or nitrogen (45 l/h) as specified. The Al₂O₃ support used was an α-Al₂O₃ support from Scientific Design. The shaped bodies obtained were crushed and used further as the 200 to 315 µm sieve fraction. The support was washed with demineralized water, filtered and dried at 150°C in a muffle furnace overnight. For preparation of the impregnation solution, one equivalent of silver oxide (Ag₂O, 16.8 g, Sigma-Aldrich, purity > 99%) was suspended in 75 ml of water. In addition, one equivalent of oxalic acid (9.0 g) was dissolved in 150 ml of demineralized water. The oxalic acid solution was added gradually to the Ag₂O suspension with continuous stirring at room temperature and then stirred for 15 min, in the course of which the colour of the suspension changed from black to brown. The suspension was then filtered, and the solids were washed with 9 ml of demineralized water and introduced into a 50 ml flask. 21 ml of demineralized water and a magnetic stirrer bar were added to the flask, which was subsequently cooled in an ice bath. In parallel, a 72% solution of ethylenediamine (Sigma-Aldrich, purity > 99%) in demineralized water was prepared (9 ml of ethylenediamine in 3 ml of demin. water). The solution was gradually added dropwise to the cooled suspension of the silver catalyst, such that there were two equivalents of EDA to one equivalent of silver. The suspension was then cooled for two further hours and stirred in the dark until a clear solution was obtained. The stirrer speed set was between 600 and 900 rpm.
The alpha-alumina support was impregnated with the clear silver-ethylenediamine solution by the incipient wetness method, by drawing the solution into the syringe with a cannula of minimum width and dripping it onto the dry catalyst support. A weight ratio of 185:400 of solution to support was set here. The dropwise addition of the solution was accompanied by permanent, manual mixing of the catalyst support.

The pale brownish solid obtained was dried in a muffle furnace under nitrogen at 100°C (ramp for 1 h to target temperature) for 4 h (muffle furnace of the N11/R type from Nabertherm). This was purged with synthetic air (14.5 l/h) or nitrogen (45 l/h) as specified. The dried powder was heated to 400°C in the same muffle furnace within 1 hour, kept at that temperature for 30 minutes, and then cooled down to room temperature. The catalyst thus obtained (catalyst B1) contains 10% by weight of silver, based on the total weight of the catalyst (determination in each case by means of ICP-OES).

For a second catalyst (catalyst B2), catalyst B1 was impregnated for a second time as described above, and catalyst B2 contained 20% by weight of silver, based on the total weight of the catalyst.

**Table 3: porosity of catalyst B2 compared to α-alumina**

| Sample | N₂ BET [m²/g] |
|---|---|
| α-Alumina | 1.175 m²/g |
| Catalyst B2 | 1.319 m²/g |

This was purged with synthetic air (14.5 l/h) or nitrogen (45 l/h) as specified.

### 4. Test results

Table 4 summarizes the catalysts and starting conditions used that were used in the test reactions with the reactor concept according to the invention. As well as the composition of the reactant stream (feed), the space-time velocity (GHSV) is reported, which may be different in each case in the region of the two catalysts. Also reported is the temperature in the region of the dehydration catalyst (Dehydrat. cat. T.) and the type of separation between first and second catalyst (Cat. separation).

Table 5 summarizes the results that have been obtained in the test reactions with the reactor concept according to the invention. As well as the conversions of the ethanol and ethene reactants, the selectivity and yield with respect to the desired ethylene oxide product (EO) is reported. In addition, the selectivity with respect to the unwanted acetic acid (AA) and C₃ to C₆ olefin by-products are reported.

**Table 4: inventive process conditions**

| **Test No.** | **Dehydrat. catalyst** | **Cat. separation** | **Epoxide catalyst** | **GHSV dehydrat. [1/h]** | **GHSV epoxid. [1/h]** | **Dehydrat. cat. T. [°C]** | **Feed O₂ [%] by vol.** | **Feed ethene [%] by vol.** | **Feed EtOH [%] by vol.** |
|---|---|---|---|---|---|---|---|---|---|
| 1 | A1 | quartz wool | B1 | 6000 | 9000 | 228 | 8.0 | 29.6 | 0.6 |
| 2 | A1 | quartz wool | B1 | 7000 | 7000 | 220 | 8.0 | 29.4 | 0.7 |
| 3 | A1 | quartz wool | B1 | 7000 | 18 000 | 232 | 8.3 | 28.0 | 0.6 |
| 4 | A1 | quartz wool | B1 | 7000 | 7000 | 194 | 8.0 | 29.4 | 0.7 |
| 5 | H-ZSM-5 (Si/Al=30) | quartz wool | B2 | 7000 | 7000 | 240 | 7.4 | 28.8 | 6.2 |
| 6 | H-ZSM-5 (Si/Al=45) | quartz wool | B2 | 7000 | 7000 | 230 | 9.0 | 0 | 10.8 |
| 7 | A1 | quartz wool | B1 | 7000 | 7000 | 220 | 18.2 | 0 | 8.4 |
| 8 | A1 | quartz wool | B1 | 7000 | 30 000 | 237 | 8.2 | 29.3 | 0.7 |
| 9 | A1 | quartz wool | B1 | 7000 | 25 000 | 248 | 8.3 | 29.4 | 0.7 |
| 10 | A2 | heated pipeline | B1 | 4246 | 4681 | 230 | 8 | 29.4 | 0.66 |
| 11 | A2 | heated pipeline | B1 | 4246 | 4681 | 230 | 8 | 29.4 | 0.66 |
| 12 | A2 | heated pipeline | B1 | 4246 | 4681 | 230 | 7.82 | 28.38 | 0.55 |
| 13 | A2 | heated pipeline | B1 | 4246 | 4681 | 230 | 7.82 | 28.38 | 0.55 |
| 14 | A2 | quartz wool | B1 | 7000 | 7000 | 235 | 7.62 | 27.98 | 0.61 |

**Table 5: results for reactor concept according to the invention**

| **Test No.** | **EtOH conversion** | **Ethene conversion** | **EO selectivity** | **Diethyl ether selectivity** | **EO yield** | **AA selectivity** | **Ethene selectivity** | **C₃-C₆ olefin selectivity** |
|---|---|---|---|---|---|---|---|---|
| 1 | 100% | 4.3% | 34.5% | 0.0% | 1.47% | 0.00% | - | - |
| 2 | 100% | 10.1% | 28.10% | 0.02% | 0.66% | 0.00% | - | - |
| 3 | 100% | 4.90% | 25.40% | 0.03% | 1.23% | 0.40% | ∼100% | 0.83% |
| 4 | 99.50% | 0.24% | 0% | 9.80% | 0% | 0% | - | 6.37% |
| 5 | 99.50% | 0% | 0% | 0.20% | 0% | 0% | - | - |
| 6 | 99.96% | 30.30% | < 1% | 0.00% | - | 0% | ∼100% | > 1% |
| 7 | 100% | 22.70% | 27% | 0.00% | 27% | 0.00% | ∼100% | 0.80% |
| 8 | 100% | 13% | 11% | 0% | 0.48% | 0.21% | ∼100% | 0.70% |
| 9 | 100% | 12.80% | 12% | 0% | 0.67% | 0.32% | ∼100% | 0.60% |
| 10 | 100% | 3.95% | 56.71% | 0.00% | 2.24% | 0.00% | >99.5% | 0.00% |
| 11 | 100% | 5.11% | 47.26% | 0.00% | 2.41% | 0.00% | >99.5% | 0.00% |
| 12 | 100% | 5.44% | 44.54% | 0.00% | 2.42% | 0.00% | >99.5% | 0.00% |
| 13 | 100% | 3.75% | 51.72% | 0.00% | 1.94% | 0.00% | >99.5% | 0.00% |
| 14 | 100% | 7.84% | 40.54% | 0.00% | 3.18% | 0.00% | - | < 0.1% |

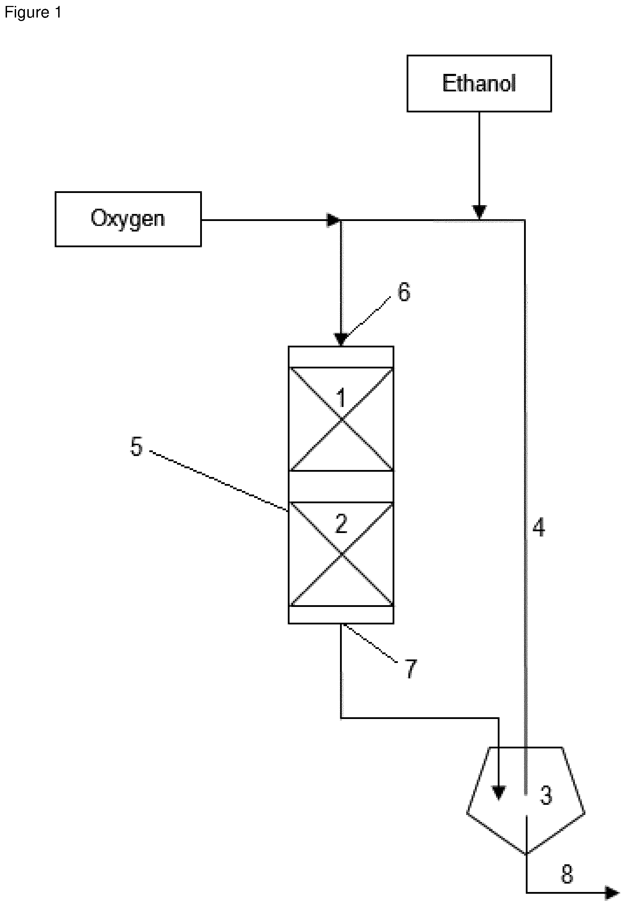

## Claims

1. Catalyst arrangement for preparation of ethylene oxide from ethanol, comprising a first catalyst [1] which is suitable for dehydration of ethanol to ethylene and is in fluid connection to a second catalyst [2] suitable for oxidation of ethylene to ethylene oxide with oxygen, **characterized in that** the first catalyst [1] is an acidic catalyst having an overall density of acidic sites of more than 1 µmol/m².

2. Catalyst arrangement according to Claim 1, **characterized in that** the density of acidic sites in the first catalyst [1] is greater than 1.2 µmol/m², preferably greater than 1.5 µmol/m².

3. Catalyst arrangement according to Claim 1 or 2, **characterized in that** the first catalyst [1] is an acidic catalyst comprising a tungsten-containing heteropolyacid or a sulfated metal oxide or an acidic zeolite.

4. Catalyst arrangement according to any of the preceding claims, **characterized in that** the first catalyst [1] is sulfated zirconium dioxide or tungstosilicic acid on an SiO₂ support.

5. Catalyst arrangement according to any of the preceding claims, **characterized in that** the second catalyst [2] comprises Ag on an alumina support.

6. Catalyst arrangement according to any of the preceding claims, **characterized in that** the first and the second catalyst [2] are in direct fluid connection.

7. Catalyst arrangement according to any of the preceding claims, **characterized in that** both the first catalyst [1] and the second catalyst [2] are in a single reactor [5].

8. Catalyst arrangement according to Claim 7, **characterized in that** the single reactor [5] is configured as a tube.

9. Catalyst arrangement according to any of the preceding claims, **characterized in that** the temperature in the region of the first catalyst [1] can be controlled independently of the temperature in the region of the second catalyst [2].

10. Catalyst arrangement according to any of the preceding claims, **characterized in that** the first catalyst [1] and/or the second catalyst [2] takes the form of a fixed catalyst bed.

11. Use of a catalyst arrangement according to any of Claims 1 to 10 for preparation of ethylene oxide from ethanol.

12. Process for preparing ethylene oxide from ethanol, wherein a gas stream comprising oxygen and ethanol is guided through a catalyst arrangement according to any of Claims 1 to 10 in order to obtain a product gas stream.

13. Process for preparing ethylene oxide from ethanol, comprising the steps of:
a) contacting ethanol with a first catalyst [1] suitable for dehydration of ethanol to ethylene to obtain ethylene,
b) contacting the ethylene from step a) with a second catalyst [2] suitable for oxidation of ethylene to ethylene oxide with oxygen,
**characterized in that** the first catalyst [1] is an acidic catalyst having an overall density of acidic sites of more than 1 µmol/m².

14. Process according to either of Claims 12 or 13, **characterized in that** the temperature in the region of the first catalyst [1] is between 100°C and 300°C, preferably between 200°C and 280°C, more preferably between 215°C and 270°C.

15. Process according to any of Claims 12 to 14, **characterized in that** the temperature in the region of the second catalyst [2] is between 150°C and 300°C, preferably between 210°C and 280°C.
